# EUROPEAN PATENT APPLICATION

(11) **EP 3 130 660 A1**
(43) Date of publication of application: **15.02.2017**
(21) Application number: 15767900.2
(22) Date of filing: 24.03.2015
(51) Int. Cl.: C12M 1/42, C12N 1/00, C12N 5/071, C12N 5/10, C12N 13/00

(54) **CELL SEPARATION DEVICE AND CELL SEPARATION METHOD**

(30) Priority: 25.03.2014 JP 2014062374
(71) Applicant: Tokyo Electron Limited, Tokyo 107-6325 (JP)
(72) Inventor: GOMI Shinichi, Tokyo 107-6325 (JP); OSHIMA Yasuhiro, Stevenage Hertfordshire SG1 2FX (GB); KAGAWA Kenichi, Tokyo 107-6325 (JP); KURAKAZU Tomoaki, Stevenage Hertfordshire SG1 2FX (GB); GOMI Hisashi, Tokyo 107-6325 (JP); OZAKI Shigenori, Tokyo 107-6325 (JP)
(74) Representative: Diehl & Partner GbR
(86) International application number: PCT/JP2015/058841
(87) International publication number: WO 2015/146952

(57) **Abstract**

A cell separation device (140) imparts oscillation from a back-face side to a cell culture film (77) to which cells adhere to the surface thereof and thereby separates a cell from the cell culture film (77). The cell separation device (140) is provided with an ultrasonic probe (141) having an oscillator (141 a) for ultrasonically oscillating, a frame (142) disposed so as to surround the oscillator (141a), and a first detection unit (143) for detecting a force applied to the frame (142) in a predetermined direction. The oscillator (141a) imparts ultrasonic oscillation selectively to a predetermined cell adhering to the surface of the cell culture film (77). The frame (142) is pushed in until the force applied to the frame (142) from the back face of the cell culture film (77) reaches a predetermined magnitude on the basis of the result of detection by the first detection unit (143), and the oscillator (141a) then ultrasonically oscillates in a state in which the frame (142) abuts the back face of the cell culture film (77).

## Description

### TECHNICAL FIELD

The present disclosure relates to a cell separation device and a cell separation method for separating cells from a cell culture film.

### BACKGROUND

There are known methods to culture cells within a culture container and to separate cells adhering to the culture container. For example, Japanese laid-open publication No. 2006-314204 (hereinafter referred to as Patent Document 1) discloses a technique in which cells are separated from a cell adhesion surface by imparting ultrasonic vibration to a culture container at the rear surface side of the cell adhesion surface. More specifically, in Patent document 1, when performing a subculture after a predetermined culture period has elapsed, a main body is inverted so that the rear-surface-side of the cell adhesion surface is oriented upward. Then, a vibrator of an ultrasonic wave generation device is brought into contact with the entire outer surface and ultrasonic vibration is imparted to the culture container, thereby separating the cells.

In addition to the uniform recovery of cells from the culture container, it is also important to selectively separate defective cells that could not be successfully cultured for any reason. The invention disclosed in Patent Document 1 is configured to recover all the cells by bringing the vibrator of the ultrasonic wave generation device into contact with the entire surface and is not capable of selectively separating the cells.

### SUMMARY

The present disclosure provides a cell separation device and a cell separation method capable of selectively separating only specified cells such as defective cells or the like by virtue of ultrasonic vibration.

According to the present disclosure, there is provided a cell separation device for imparting vibration to a cell culture film having a front surface to which cells adhere, from a rear surface side of the cell culture film, and consequently separating the cells from the cell culture film, including:
an ultrasonic probe having an ultrasonically-vibrating vibrator,
wherein the vibrator selectively making contact with a portion of a rear surface of the cell culture film is ultrasonically vibrated.

The cell separation device according to the present disclosure further includes:
a second detection part configured to detect a force applied from the rear surface of the cell culture film to the vibrator or a distance between the vibrator and the rear surface of the cell culture film,
wherein based on a detection result obtained by the second detection part, the vibrator is pressed against the rear surface of the cell culture film until the force applied from the rear surface of the cell culture film to the vibrator reaches a specified magnitude, and then the vibrator making contact with the rear surface of the cell culture film is ultrasonically vibrated.

The cell separation device according to the present disclosure may further include:
a cell-culture-film-side frame disposed to surround a range becoming a separation operation target or a vibrator-side frame disposed to surround the vibrator,
wherein the vibrator making contact with the rear surface of the cell culture film may be ultrasonically vibrated to separate the cells from the cell culture film, and
the cell-culture-film-side frame or the vibrator-side frame may always make contact with the rear surface of the cell culture film while separating the cells from the cell culture film.

The cell separation device according to the present disclosure may further include:
a vibrator-side frame disposed to surround the vibrator,
wherein the vibrator-side frame may be first pressed against the rear surface of the cell culture film, and then the vibrator making contact with the rear surface of the cell culture film may be ultrasonically vibrated in a state in which the vibrator-side frame makes contact with the rear surface of the cell culture film.

The cell separation device according to the present disclosure may further include:
a first detection part configured to detect a force applied from the rear surface of the cell culture film to the vibrator-side frame or a distance between the vibrator-side frame and the rear surface of the cell culture film,
wherein based on a detection result obtained by the first detection part, the vibrator-side frame may be pressed against the rear surface of the cell culture film until the force applied from the rear surface of the cell culture film to the vibrator-side frame reaches a specified magnitude, and then the vibrator making contact with the rear surface of the cell culture film may be ultrasonically vibrated in a state in which the vibrator-side frame makes contact with the rear surface of the cell culture film.

In the cell separation device according to the present disclosure, the ultrasonic probe and the vibrator-side frame may be connected to each other through an elastic member,
a cell-culture-film-side end portion of the vibrator may be accommodated inside the vibrator-side frame when the frame does not make contact with the cell culture film, and
the cell-culture-film-side end portion of the vibrator may protrude out of the vibrator-side frame as the vibrator-side frame is pressed against the cell culture film.

In the cell separation device according to the present disclosure, the first detection part or the second detection part may be a contact sensor, a proximity sensor or a force sensor.

In the cell separation device according to the present disclosure, the vibrator-side frame may include a first frame making contact with the rear surface of the cell culture film and a second frame positioned at a side of a base end portion of the first frame, and
an elastic modulus of the first frame may be larger than an elastic modulus of the second frame.

In the cell separation device according to the present disclosure, the vibrator-side frame may include a first frame making contact with the rear surface of the cell culture film and a second frame positioned at a side of a base end portion of the first frame, and
an elastic modulus of the first frame may be smaller than an elastic modulus of the second frame.

The cell separation device according to the present disclosure may further include:
one elevator part configured to simultaneously and vertically drive the ultrasonic probe and the vibrator-side frame with respect to the rear surface of the cell culture film.

According to a first aspect of the present disclosure, there is provided a cell separation device, including:
an ultrasonic probe having an ultrasonically-vibrating vibrator; and
a frame disposed to surround the vibrator,
wherein the frame is first pressed against a rear surface of a cell culture film, and then the vibrator making contact with the rear surface of the cell culture film is ultrasonically vibrated in a state in which the frame makes contact with the rear surface of the cell culture film.

According to a second aspect of the present disclosure, there is provided a cell separation device, including:
an ultrasonic probe having an ultrasonically-vibrating vibrator;
a frame disposed to surround the vibrator; and
a first detection part configured to detect a force applied from a rear surface of a cell culture film to the frame or a distance between the frame and the rear surface of the cell culture film,
wherein based on a detection result obtained by the first detection part, the frame is pressed against the rear surface of the cell culture film until the force applied from the rear surface of the cell culture film to the frame reaches a specified magnitude, and then the vibrator making contact with the rear surface of the cell culture film is ultrasonically vibrated in a state in which the frame makes contact with the rear surface of the cell culture film.

In the cell separation device according to the first aspect, the second aspect, a fourth aspect, the fifth aspect and the sixth aspect of the present disclosure, the ultrasonic probe and the vibrator-side frame may be connected to each other through an elastic member,
a cell-culture-film-side end portion of the vibrator may be accommodated inside the vibrator-side frame when the frame does not make contact with the cell culture film, and
the cell-culture-film-side end portion of the vibrator may protrude out of the vibrator-side frame as the vibrator-side frame is pressed against the cell culture film.

In the cell separation device according to the second aspect, a third aspect, the fourth aspect, the fifth aspect and the sixth aspect of the present disclosure, the first detection part or the second detection part may be a contact sensor, a proximity sensor or a force sensor,
in the case where the first detection part or the second detection part is a contact sensor, the pressing of the frame against the rear surface of the cell culture film may be stopped at a time point at which the frame makes contact with the contact sensor,
in the case where the first detection part or the second detection part is a proximity sensor, the pressing of the frame against the rear surface of the cell culture film may be stopped at a time point at which the frame has a predetermined distance from the proximity sensor, and
in the case where the first detection part or the second detection part is a force sensor, the pressing of the frame against the rear surface of the cell culture film may be stopped at a time point at which a force applied from the frame to the force sensor reaches a specified magnitude.

In the cell separation device according to the first aspect, the second aspect, the fourth aspect, the fifth aspect and the sixth aspect of the present disclosure, the frame may include a first frame making contact with the rear surface of the cell culture film and a second frame positioned at a side of the first detection part with respect to the first frame, and
an elastic modulus of the first frame may be larger than an elastic modulus of the second frame.

In the cell separation device according to the first aspect of the present disclosure, the ultrasonic probe and the frame may be connected to each other through a plurality of elastic members, and
the respective elastic members may be disposed at regular intervals.

According to the third aspect of the present disclosure, there is provided a cell separation device, including:
an ultrasonic probe having an ultrasonically-vibrating vibrator; and
a second detection part configured to detect a force applied from a rear surface of a cell culture film to the vibrator or a distance between the vibrator and the rear surface of the cell culture film,
wherein based on a detection result obtained by the second detection part, the vibrator is pressed against the rear surface of the cell culture film until the force applied from the rear surface of the cell culture film to the vibrator reaches a specified magnitude, and then the vibrator making contact with the rear surface of the cell culture film is ultrasonically vibrated.

According to the fourth aspect of the present disclosure, there is provided a cell separation device, including:
an ultrasonic probe having an ultrasonically-vibrating vibrator;
a frame disposed to surround the vibrator; and
a second detection part configured to detect a force applied from a rear surface of a cell culture film to the vibrator or a distance between the vibrator and the rear surface of the cell culture film,
wherein based on a detection result obtained by the second detection part, the vibrator is pressed against the rear surface of the cell culture film until the force applied from the rear surface of the cell culture film to the vibrator reaches a specified magnitude, and then the vibrator making contact with the rear surface of the cell culture film is ultrasonically vibrated in a state in which the frame makes contact with the rear surface of the cell culture film.

According to the fifth aspect of the present disclosure, there is provided a cell separation device, including:
an ultrasonic probe having an ultrasonically-vibrating vibrator; and
a frame held on a rear surface of a cell culture film or bonded to the rear surface of the cell culture film,
wherein cells are separated from the cell culture film as the vibrator making contact with the rear surface of the cell culture film is ultrasonically vibrated, and
the frame always makes contact with the rear surface of the cell culture film while separating the cells from the cell culture film.

According to the sixth aspect of the present disclosure, there is provided a cell separation device, including:
an ultrasonic probe having an ultrasonically-vibrating vibrator;
a frame disposed to surround a range becoming a separation operation target; and
a second detection part configured to detect a force applied from a rear surface of a cell culture film to the vibrator or a distance between the vibrator and the rear surface of the cell culture film,
wherein based on a detection result obtained by the second detection part, the vibrator is pressed against the rear surface of the cell culture film until the force applied from the rear surface of the cell culture film to the vibrator reaches a specified magnitude, and then cells are separated from the cell culture film as the vibrator making contact with the rear surface of the cell culture film is ultrasonically vibrated, and
the frame always makes contact with the rear surface of the cell culture film while separating the cells from the cell culture film.

In the cell separation device according to the first aspect, the second aspect, the fourth aspect, the fifth aspect and the sixth aspect of the present disclosure, the frame may be configured with a plurality of members differing in elastic modulus from one another.

In the cell separation device according to the second aspect, the third aspect, the fourth aspect and the sixth aspect of the present disclosure, the first detection part and the second detection part may be arbitrarily selected by a person of knowledge and may be, for example, a contact sensor, a proximity sensor or a force sensor.

The cell separation device according to the present disclosure may further include:
an elevator part configured to vertically drive the ultrasonic probe with respect to the rear surface of the cell culture film in response to position control of the ultrasonic probe.

In the cell separation device according to the present disclosure, a cell-culture-film-side end portion of the vibrator may be subjected to a knurling process.

In the cell separation device according to the present disclosure, the cells separated from the cell culture film may be defective cells not required in culture or subculture.

In the cell separation device according to the present disclosure, the cells adhering to the front surface of the cell culture film may be iPS cells or differentiated cells.

According to the present disclosure, there is provided a cell separation method for imparting vibration to a cell culture film having a front surface to which cells adhere, from a rear surface side of the cell culture film, and consequently separating the cells from the cell culture film, including:
a step of ultrasonically vibrating a vibrator which selectively makes contact with a portion of a rear surface of the cell culture film,
wherein the vibrator is configured to selectively impart ultrasonic vibration to specified cells adhering to the front surface of the cell culture film.

The cell separation method according to the present invention may further include:
a step of bringing the vibrator into contact with the rear surface of the cell culture film, and
a step of, based on a detection result obtained by a second detection part for detecting a force applied from the rear surface of the cell culture film to the vibrator or a distance between the vibrator and the rear surface of the cell culture film, pressing the vibrator against the rear surface of the cell culture film until the force applied from the rear surface of the cell culture film to the vibrator reaches a specified magnitude.

The cell separation method according to the present invention may further include:
a step of bringing a vibrator-side frame or a cell-culture-film-side frame into contact with the rear surface of the cell culture film;
a step of bringing the vibrator into contact with the rear surface of the cell culture film, and
a step of, based on a detection result obtained by a second detection part for detecting a force applied from the rear surface of the cell culture film to the vibrator or a distance between the vibrator and the rear surface of the cell culture film, pressing the vibrator against the rear surface of the cell culture film until the force applied from the rear surface of the cell culture film to the vibrator reaches a specified magnitude,
wherein the step of ultrasonically vibrating the vibrator which makes contact with the rear surface of the cell culture film may be performed in a state in which the vibrator-side frame or the cell-culture-film-side frame makes contact with the rear surface of the cell culture film.

The cell separation method according to the present invention may further include:
a step of disposing a cell-culture-film-side frame to surround a range becoming a separation operation target;
a step of pressing the vibrator against the rear surface of the cell culture film; and
a step of, based on a detection result obtained by a second detection part for detecting a force applied from the rear surface of the cell culture film to the vibrator or a distance between the vibrator and the rear surface of the cell culture film, pressing the vibrator against the rear surface of the cell culture film until the force applied from the rear surface of the cell culture film to the vibrator reaches a specified magnitude,
wherein the step of ultrasonically vibrating the vibrator which makes contact with the rear surface of the cell culture film may be performed in a state in which the cell-culture-film-side frame makes contact with the rear surface of the cell culture film.

The cell separation method according to the present invention may further include:
a step of bringing a vibrator-side frame into contact with the rear surface of the cell culture film;
a step of, based on a detection result obtained by a first detection part for detecting a force applied from the rear surface of the cell culture film to the vibrator-side frame or a distance between the vibrator-side frame and the rear surface of the cell culture film, pressing the vibrator-side frame against the rear surface of the cell culture film until the force applied from the rear surface of the cell culture film to the vibrator-side frame reaches a specified magnitude; and
a step of pressing the vibrator against the rear surface of the cell culture film,
wherein the step of ultrasonically vibrating the vibrator which makes contact with the rear surface of the cell culture film may be performed in a state in which the vibrator-side frame makes contact with the rear surface of the cell culture film.

According to the first aspect of the present disclosure, there is provided a cell separation method, including:
a step of bringing a frame into contact with a rear surface of a cell culture film;
a step of pressing a vibrator against the rear surface of the cell culture film; and
a step of causing the vibrator making contact with the rear surface of the cell culture film to ultrasonically vibrate in a state in which the frame makes contact with the rear surface of the cell culture film,
wherein the vibrator is configured to selectively impart ultrasonic vibration to specified cells adhering to a front surface of the cell culture film.

According to the second aspect of the present disclosure, there is provided a cell separation method, including:
a step of bringing a frame into contact with a rear surface of a cell culture film;
a step of, based on a detection result obtained by a first detection part for detecting a force applied from the rear surface of the cell culture film to the frame or a distance between the frame and the rear surface of the cell culture film, pressing the frame against the rear surface of the cell culture film until the force applied from the rear surface of the cell culture film to the frame reaches a specified magnitude;
a step of pressing the vibrator against the rear surface of the cell culture film; and
a step of causing the vibrator making contact with the rear surface of the cell culture film to ultrasonically vibrate in a state in which the frame makes contact with the rear surface of the cell culture film,
wherein the vibrator is configured to selectively impart ultrasonic vibration to specified cells adhering to a front surface of the cell culture film.

According to the third aspect of the present disclosure, there is provided a cell separation method, including:
a step of bringing a vibrator into contact with a rear surface of a cell culture film;
a step of bringing the vibrator into contact with a rear surface of the cell culture film;
a step of, based on a detection result obtained by a second detection part for detecting a force applied from the rear surface of the cell culture film to the vibrator or a distance between the vibrator and the rear surface of the cell culture film, pressing the vibrator against the rear surface of the cell culture film until the force applied from the rear surface of the cell culture film to the vibrator reaches a specified magnitude; and
a step of ultrasonically vibrating the vibrator which makes contact with the rear surface of the cell culture film,
wherein the vibrator is configured to selectively impart ultrasonic vibration to specified cells adhering to a front surface of the cell culture film.

According to the fourth aspect of the present disclosure, there is provided a cell separation method, including:
a step of bringing a frame into contact with a rear surface of a cell culture film;
a step of bringing a vibrator into contact with the rear surface of the cell culture film;
a step of, based on a detection result obtained by a second detection part for detecting a force applied from the rear surface of the cell culture film to the vibrator or a distance between the vibrator and the rear surface of the cell culture film, pressing the vibrator against the rear surface of the cell culture film until the force applied from the rear surface of the cell culture film to the vibrator reaches a specified magnitude; and
a step of causing the vibrator making contact with the rear surface of the cell culture film to ultrasonically vibrate in a state in which the frame makes contact with the rear surface of the cell culture film,
wherein the vibrator is configured to selectively impart ultrasonic vibration to specified cells adhering to a front surface of the cell culture film.

According to the fifth aspect of the present disclosure, there is provided a cell separation method, including:
a step of disposing a frame to surround a range becoming a separation operation target;
a step of pressing a vibrator against a rear surface of a cell culture film; and
a step of causing the vibrator making contact with the rear surface of the cell culture film to ultrasonically vibrate in a state in which the frame makes contact with the rear surface of the cell culture film,
wherein the vibrator is configured to selectively impart ultrasonic vibration to specified cells adhering to a front surface of the cell culture film.

According to the sixth aspect of the present disclosure, there is provided a cell separation method, including:
a step of disposing a frame to surround a range becoming a separation operation target;
a step of bringing a vibrator against a rear surface of a cell culture film;
a step of, based on a detection result obtained by a second detection part for detecting a force applied from the rear surface of the cell culture film to the vibrator or a distance between the vibrator and the rear surface of the cell culture film, pressing the vibrator against the rear surface of the cell culture film until the force applied from the rear surface of the cell culture film to the vibrator reaches a specified magnitude; and
a step of causing the vibrator making contact with the rear surface of the cell culture film to ultrasonically vibrate in a state in which the frame makes contact with the rear surface of the cell culture film,
wherein the vibrator is configured to selectively impart ultrasonic vibration to specified cells adhering to a front surface of the cell culture film.

According to the present disclosure, the vibrator selectively imparts ultrasonic vibration to the specified cells adhering to the front surface of the cell culture film. Thus, it is possible to realize the selective separation of specified cells such as defective cells or the like by virtue of ultrasonic vibration, which was difficult to realize in the technique disclosed in Patent Document 1.

In the first aspect, the second aspect, the fourth aspect, the fifth aspect and the sixth aspect of the present disclosure, the vibrator is ultrasonically vibrated in a state in which the frame supports the rear surface of the cell culture film around the vibrator. Since the frame makes contact with the rear surface of the cell culture film at the outer side of the peripheral edge of the vibrator in this way, it is possible to prevent the vibration of the cell culture film attributable to the vibration of the vibrator from spreading to the outer side of the peripheral edge of the frame. Thus, it is possible to limit the influence of the vibration generated by the vibrator to the specified target cells such as defective cells or the like as far as possible. This makes it possible to prevent good cells adjoining the specified cells such as defective cells or the like from being separated by mistake.

In the second aspect, the third aspect, the fourth aspect and the sixth aspect of the present disclosure, the mechanism for detecting the force by which the ultrasonic probe is pressed against the cell culture film is provided by the first detection part installed in the frame or the second detection part installed in the vibrator. As the vibrator is more strongly pressed against the rear surface of the cell culture film, the vibration is propagated farther. Thus, it is highly likely that the cells other than the specified cells are separated. Since the pressing force can be arbitrarily set by the first detection part or the second detection part, it is possible to limit the influence of the vibration generated by the vibrator to the specified target cells such as defective cells or the like as far as possible. This makes it possible to prevent good cells adjoining the specified cells such as defective cells or the like from being separated by mistake.

### BRIEF DESCRIPTION OF THE DRAWINGS

**FIG. 1** is a schematic top plane view illustrating the configuration of an automatic culture system according to an embodiment of the present disclosure.
**FIG. 2** is a control block diagram of the automatic culture system according to an embodiment of the present disclosure.
**FIG. 3** is a control block diagram of a cell inspection removal part according to an embodiment of the present disclosure.
**FIG. 4A** is a perspective view illustrating a state in which an airtight container is held in a cell separation mechanism according to the present disclosure.
**FIG. 4B** is a view illustrating a cross section of the cell separation mechanism illustrated in **FIG. 4A**.
**FIG. 5** is a perspective view of a cell separation device according to a second aspect of the present disclosure.
**FIG. 6** is a schematic sectional view illustrating a state in which the cell separation device according to the second aspect of the present disclosure makes contact with the rear surface of a film. Since the cell separation device illustrated in **FIG. 6** is shown in a simplified manner, the shape thereof differs from the shape of the cell separation device illustrated in **FIG. 5****.**
**FIG. 7A** is a schematic sectional view corresponding to **FIG. 6** and illustrates a state in which the cell separation device making contact with the rear surface of the film is being pushed toward the film. In this case, a contact sensor is used as a first detection part.
**FIG. 7B** is a schematic sectional view corresponding to **FIG. 6** and illustrates a state in which the cell separation device making contact with the rear surface of the film is being pushed toward the film. In this case, a contact sensor is used as a first detection part.
**FIG. 7C** is a schematic sectional view corresponding to **FIG. 6** and illustrates a state in which the cell separation device making contact with the rear surface of the film is being pushed toward the film. In this case, a contact sensor is used as a first detection part.
**FIG. 8** is a perspective view of a cell separation device according to a modification of the second aspect of the present disclosure.
**FIG. 9** is a schematic sectional view illustrating a state in which a cell separation device according to a fifth aspect of the present disclosure makes contact with the rear surface of a film.
**FIG. 10** is a graph in which the up-down direction movement distance of an ultrasonic probe is indicated in a horizontal axis and the average output power of the ultrasonic probe is indicated in a vertical axis.
**FIG. 11** is a perspective view illustrating the film-side end portion of a vibrator subjected to a knurling process.
**FIG. 12** is a perspective view of a cell separation device according to a modification in which the elastic modulus of a first frame is smaller than the elastic modulus of a second frame.

### DETAILED DESCRIPTION

The cell separation device according to the present embodiment can be used with respect to all kinds of cells having an adhering property (bonding property). The cell separation device can be used in culturing different cells including pluripotent stem cells such as (human) iPS cells, (human) ES cells or the like, chondrocytes such as bone marrow stromal cells (MSC) or the like, dendritic cells, and so forth. In the present embodiment, descriptions will be made using an automatic culture system which automatically cultures iPS cells. However, it should be noted that this is nothing more than one example.

### [Overall Configuration]

Next, descriptions will be made on the device configuration of an automatic culture system according to the present embodiment.

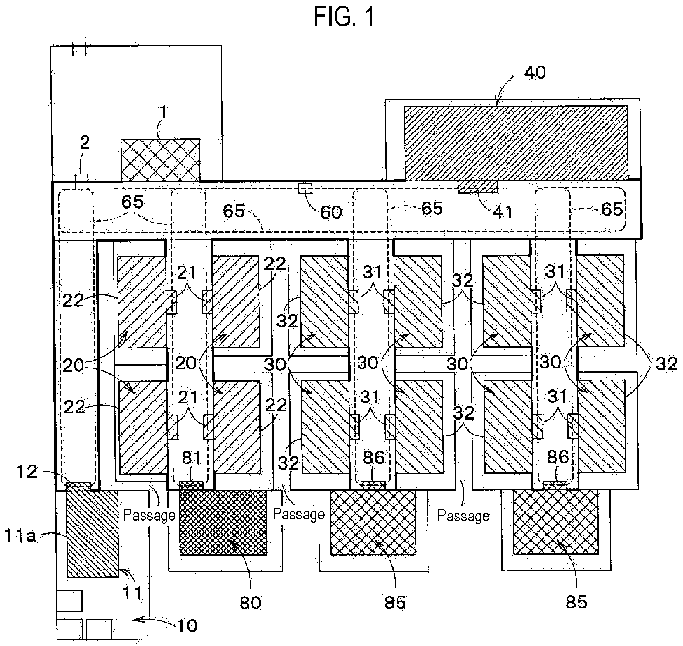

As illustrated in **FIG. 1**, the automatic culture system according to the present embodiment includes a raw material storage device 10 which stores raw material cells, a container transfer part 60 which transfers a first airtight container (not shown) that accommodates cells in a sealed state, and automatic culture devices 20 and 30 which receive the first airtight container transferred by the container transfer part 60, take out second airtight containers 75 formed of OptiCell (trademark) from the first airtight container and culture cells within the taken-out second airtight containers 75.

In the present embodiment, as described above, an aspect using iPS cells is described. Thus, the raw material storage device 10 includes an iPS cell establishing device 11 which establishes iPS cells. In addition, the raw material storage device 10 includes a unit thermostatic bath, a centrifuge, an automatic blood cell counting device, an automatic magnetic cell separator, a flow cytometer, a gene introduction device, and so forth.

The automatic culture devices 20 and 30 of the present embodiment include a plurality of (four, in the aspect illustrated in **FIG. 1**) iPS-cell automatic culture devices 20 which automatically culture iPS cells and a plurality of (eight, in the aspect illustrated in **FIG. 1**) differentiated-cell automatic culture devices 30 which automatically culture differentiated cells differentiated from the iPS cells. In the present embodiment, when merely saying "automatic culture devices", it refers to the iPS-cell automatic culture devices 20, the differentiated-cell automatic culture devices 30, or both of the iPS-cell automatic culture devices 20 and the differentiated-cell automatic culture devices 30. Furthermore, in the present embodiment, when merely saying "cells", it refers to raw material cells, such as somatic cells which become the source of iPS cells or the like, iPS cells, differentiated cells, or two or all of the raw material cells, the iPS cells and the differentiated cells, unless otherwise specified.

In the present embodiment, in addition to the first airtight container, there are employed the second airtight containers 75 mentioned above. The first airtight container includes a plurality of racks for mounting the second airtight containers 75. The second airtight containers 75 are mounted in the respective racks. In a state in which the second airtight containers 75 are accommodated within the first airtight container, the first airtight container is transferred by the container transfer part 60. The second airtight containers 75 may accommodate not only the cells but also a liquid medium, a chemical and the like among the materials which will be described later.

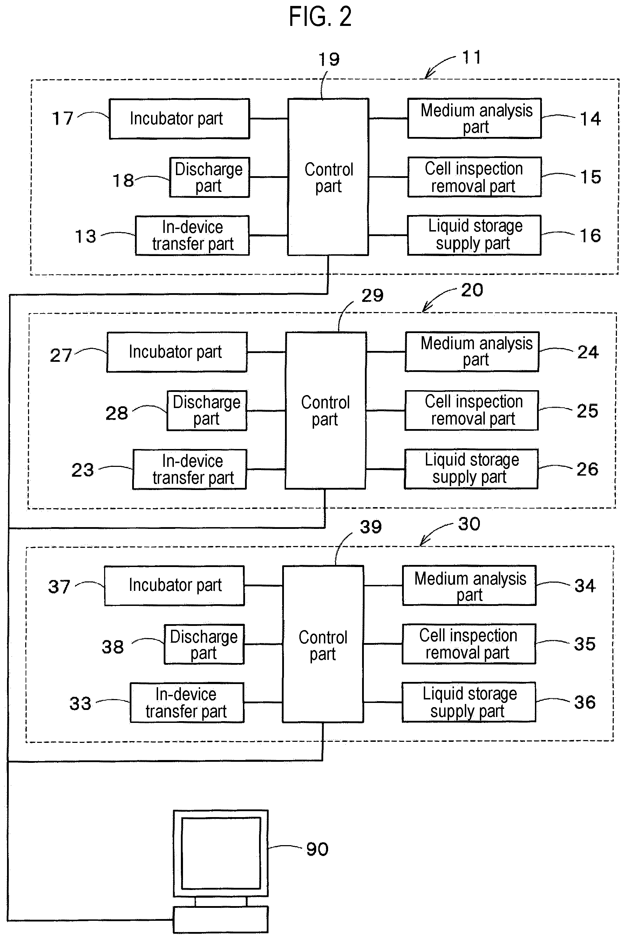

The iPS-cell automatic culture device 20 includes a housing 22 illustrated in **FIG. 1**, a medium analysis part 24 which analyzes liquid culture medium components that vary with the culture of iPS cells, a cell inspection removal part 25 which inspects the iPS cells and performs removal of the iPS cells having a bad state, a liquid storage supply part 26 which stores and supplies a liquid including a liquid culture medium or a proteolytic enzyme, performs a pre-treatment before the iPS cells are seeded, seeds the iPS cells and recovers the iPS cells, an incubator part 27 which holds the second airtight container 75 and automatically adjusts one or all of a temperature, a humidity and a gas concentration, and a discharge part 28 for discharging downward from the housing 22 a waste liquid including a used liquid culture medium, a used cleaning liquid, a used reagent or the like used within the iPS-cell automatic culture device 20, all of which are illustrated in **FIG. 2****.** Furthermore, the iPS-cell automatic culture device 20 includes an in-device transfer part 23 which transfers the second airtight containers 75 and the like within the device. Moreover, the liquid storage supply part 26 described above has a function of inverting upside down the second airtight containers 75. Incidentally, in the case where the second airtight container 75 is used as in the present embodiment, the humidity within the incubator part 27 may not be particularly managed. It is therefore possible to simplify the management of a cell culture environment. By employing the second airtight container 75, there is no fear that contamination from the air occurs. Furthermore, the transfer becomes easy.

The liquid storage supply part 26 described above appropriately supplies a liquid culture medium from an inlet into the second airtight container 75, thereby automatically replacing an old liquid culture medium existing within the second airtight container 75 with a new liquid culture medium. Based on the information of the iPS cells acquired, the cell inspection removal part 25 selectively separates defective iPS cells from an ECM (Extracellular Matrix) coated on the surface of a film 77 of the second airtight container 75. Thereafter, a liquid culture medium is supplied from the inlet into the second airtight container 75, whereby floating defective iPS cells are pushed out from the second airtight container 75 through an outlet. As a method of selectively separating the iPS cells accommodated within the second airtight container 75, in the present embodiment, ultrasonic vibration is imparted to the rear surface of the film 77.

Furthermore, the liquid storage supply part 26 appropriately supplies a proteolytic enzyme from the inlet into the second airtight container 75, thereby separating the iPS cells from the ECM coated on the surface of the film 77 of the second airtight container 75. Thereafter, a liquid culture medium is supplied from the inlet into the second airtight container 75, whereby floating iPS cells are pushed out from the second airtight container 75 through the outlet. The iPS cells thus pushed out are diluted into a suspension and are then accommodated (seeded) within a plurality of other second airtight containers 75. In this way, the iPS-cell automatic culture device 20 automatically performs the subculture of the iPS cells.

The internal temperature of the iPS-cell automatic culture device 20 is adjusted by the incubator part 27 so that the internal temperature becomes, for example, about 37 degrees C. Furthermore, the gas concentration within the iPS-cell automatic culture device 20 is adjusted by appropriately adding CO₂ to the air using the incubator part 27. If necessary, the humidity may be adjusted by the incubator part 27 so as to become about 100%.

The differentiated-cell automatic culture device 30 includes a housing 32 illustrated in **FIG. 1**, a medium analysis part 34 which analyzes liquid culture medium components that vary with the culture of differentiated cells, a cell inspection removal part 35 which inspects the differentiated cells and performs removal of the differentiated cells having a bad state, a liquid storage supply part 36 which stores and supplies a liquid and the like including a liquid culture medium or a proteolytic enzyme, performs a pre-treatment before the differentiated cells are seeded, seeds the differentiated cells and recovers the differentiated cells, an incubator part 37 which holds the second airtight container 75 and automatically adjusts one or all of a temperature, a humidity and a gas concentration, and a discharge part 38 for discharging downward from the housing 32 a waste liquid including a used liquid culture medium, a used cleaning liquid, a used reagent or the like used within the differentiated-cell automatic culture device 30, all of which are illustrated in **FIG. 2****.** Furthermore, the differentiated cell automatic culture device 30 includes an in-device transfer part 33 which transfers the second airtight containers 75 and the like within the device. In the case where the second airtight container 75 is used as described above, the humidity within the incubator part 37 may not be particularly managed. It is therefore possible to simplify the management of a cell culture environment. Moreover, the liquid storage supply part 36 has a function of inverting upside down the second airtight containers 75.

The liquid storage supply part 36 described above appropriately supplies a liquid culture medium from the inlet into the second airtight container 75, thereby automatically replacing an old liquid culture medium existing within the second airtight container 75 with a new liquid culture medium. Based on the information of the differentiated cells acquired, the cell inspection removal part 35 selectively separates defective differentiated cells from an ECM (Extracellular Matrix) coated on the surface of the film 77 of the second airtight container 75. Thereafter, a liquid culture medium is supplied from the inlet into the second airtight container 75, whereby floating defective differentiated cells are pushed out from the second airtight container 75 through the outlet. As a method of selectively separating the differentiated cells accommodated within the second airtight container 75, in the present embodiment, ultrasonic vibration is imparted to the rear surface of the film 77.

Furthermore, the liquid storage supply part 36 appropriately supplies a proteolytic enzyme from the inlet into the second airtight container 75, thereby separating the differentiated cells from the ECM coated on the surface of the film 77 of the second airtight container 75. Thereafter, a liquid culture medium is supplied from the inlet into the second airtight container 75, whereby floating differentiated cells are pushed out from the second airtight container 75 through the outlet. The differentiated cells thus pushed out are diluted into a suspension and are then accommodated (seeded) within a plurality of other second airtight containers 75. In this way, the differentiated-cell automatic culture device 30 automatically performs the subculture of the differentiated cells.

The internal temperature of the differentiated-cell automatic culture device 30 is adjusted by the incubator part 37 so that the internal temperature becomes, for example, about 37 degrees C. Furthermore, the gas concentration within the differentiated-cell automatic culture device 30 is adjusted by appropriately adding CO₂ to the air using the incubator part 37. Moreover, the liquid storage supply part 36 of the differentiated-cell automatic culture device 30 may supply a liquid culture medium including a differentiation inducing factor when inducing differentiation. If necessary, the humidity may be adjusted by the incubator part 37 so as to become about 100%.

As illustrated in **FIG. 2**, the iPS-cell automatic culture device 20 includes a control part 29 connected to the medium analysis part 24, the cell inspection removal part 25, the liquid storage supply part 26, the incubator part 27, the discharge part 28 and the in-device transfer part 23 so as to make communication therewith and configured to control them. The control part 29 manages a status, a log and a culture schedule with respect to the iPS cell automatic culture device 20 and performs a user interface function. Furthermore, the differentiated-cell automatic culture device 30 includes a control part 39 connected to the medium analysis part 34, the cell inspection removal part 35, the liquid storage supply part 36, the incubator part 37, the discharge part 38 and the in-device transfer part 33 so as to make communication therewith and configured to control them. The control part 39 manages a status, a log and a culture schedule with respect to the differentiated cell automatic culture device 30 and performs a user interface function.

The iPS cell establishing device 11 described above is similar in configuration to the iPS-cell automatic culture device 20 and the differentiated-cell automatic culture device 30. That is to say, the iPS cell establishing device 11 includes a housing 11 a illustrated in **FIG. 1**, a medium analysis part 14 which analyzes a liquid culture medium, a cell inspection removal part 15 which inspects the raw material cells and performs removal of the raw material cells having a bad state, a liquid storage supply part 16 which stores and supplies a liquid and the like including a liquid culture medium or a proteolytic enzyme, an incubator part 17 which automatically adjusts one or all of a temperature, a humidity and a gas concentration within the housing 11a, and a discharge part 18 for discharging downward from the housing 11a a waste liquid including a used liquid culture medium, a used cleaning liquid, a used reagent or the like used within the iPS cell establishing device 11, all of which are illustrated in **FIG. 2****.** The iPS cell establishment device 11 further includes an in-device transfer part 13 which transfers the second airtight containers 75 and the like within the device. Furthermore, the iPS cell establishing device 11 includes a control part 19 connected to the medium analysis part 14, the cell inspection removal part 15, the liquid storage supply part 16, the incubator part 17, the discharge part 18 and the in-device transfer part 13 so as to make communication therewith and configured to control them. The respective control parts 19, 29 and 39 are connected to an external device 90 such as, e.g., a personal computer or the like.

The container transfer part 60 of the present embodiment includes a holding portion which holds the first airtight container such that the first airtight container is suspended downward. The container transfer part 60 is configured to move along a rail 65 provided in a ceiling.

As illustrated in **FIG. 1**, the iPS-cell automatic culture device 20 includes a loading part 21 configured to load the second airtight container 75 from the first airtight container. The loading part 21 may include a cell loading/unloading part (not shown) for loading the iPS cells accommodated in the second airtight container 75 and for unloading the cultured iPS cells, and a material loading part (not shown) for loading the materials accommodated within the second airtight container 75. Similarly, as illustrated in **FIG. 1**, the differentiated-cell automatic culture device 30 includes a loading part 31 configured to load the second airtight container 75 from the first airtight container. The loading part 31 may include a cell loading/unloading part (not shown) for loading the iPS cells accommodated in the second airtight container 75 and for unloading the cultured differentiated cells, and a material loading part (not shown) for loading materials accommodated within the second airtight container 75. In the present embodiment, the materials include a liquid culture medium, a reagent, a cleaning liquid, a culture plate, a vial, a filter, a needle, and so forth. Furthermore, as illustrated in **FIG. 1**, the iPS cell establishing device 11 includes a loading part 12 configured to load the first airtight container.

As illustrated in **FIG. 1**, the automatic culture system of the present embodiment includes a sterilizing device 1 for sterilizing the interior of the first airtight container, an iPS cell analysis device 80 which receives the iPS cells cultured in the iPS cell automatic culture device 20 from the loading parts 81 at a predetermined timing and inspects the iPS cells, a differentiated cell analysis device 85 which receives the differentiated cells cultured in the differentiated cell automatic culture device 30 from the loading parts 86 at a predetermined timing and inspects the differentiated cells, and a freezing storage device 40 which receives the iPS cells, the differentiated cells or both cultured in the automatic culture devices 20 and 30 from the loading part 41, and freezes and stores the iPS cells, the differentiated cells or both. There may be provided a plurality of freezing storage devices 40. The entire room may be cooled and the room per se may serve as a freezer. In the case where there is provided a plurality of freezing storage devices 40 in the room or in the case where the room per se serves as a freezer, a rail 65 may be provided in the ceiling of the room so that the container transfer part 60 can move along the rail 65.

One example of the sterilizing device 1 described above may include a sterilizing device which sterilizes the interior of the first airtight container by supplying a sterilizing gas such as a hydrogen peroxide gas or a high-temperature gas into the first airtight container. Another example of the sterilizing device 1 may include a sterilizing device which sterilizes the interior of the first airtight container by irradiating, for example, γ rays or ultraviolet rays from the outside while keeping the first airtight container in a sealed state. In addition, before the first airtight container is loaded from the outside, the interior of the first airtight container may be sterilized using, for example, γ rays or ultraviolet rays. There may be a case where the liquid culture medium or the like contains protein or the like which is broken by γ rays or ultraviolet rays. In this case, it is desirable that sterilization is performed by a sterilizing gas such as a hydrogen peroxide gas, a high-temperature gas or the like.

### [Cell Inspection Removal Parts 25 and 35]

The cell inspection removal parts 25 and 35 used in the iPS cell automatic culture device 20 and the differentiated cell automatic culture device 30 of the present embodiment have the same device configuration. Thus, in the following descriptions, the iPS cells and the differentiated cells will be generically referred to as "cells". The cell inspection removal part 25 used in the iPS cell automatic culture device 20 and the cell inspection removal part 35 used in the differentiated cell automatic culture device 30 will be described together.

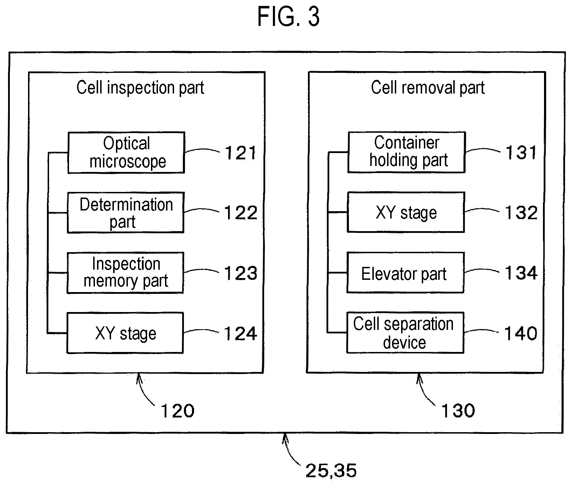

As illustrated in **FIG. 3**, each of the cell inspection removal parts 25 and 35 of the present embodiment includes a cell inspection part 120 which takes a picture of colonies of cells and determines the quality of the colonies based on the situation of the colonies of cells, and a cell removal part 130 which separates defective cells detected by the cell inspection part 120.

As illustrated in **FIG. 3**, the cell inspection part 120 includes an optical microscope 121, such as a phase-contrast microscope or the like, for observing the colonies of cells, a determination part 122 which recognizes the positions of the colonies of cells based on the picture obtained by the optical microscope 121 and automatically determines the quality of each of the colonies, an inspection memory part 123 which, if a colony of defective cells exists, stores the determination result of the position or the like of the colony of defective cells obtained by the determination part 122, and an XY stage 124 which freely moves the held second airtight container 75 in the horizontal direction. The quality of each of the colonies of cells can be determined based on, for example, a nuclear staining pattern obtained from a nuclear staining image of iPS cells or a density of iPS cells.

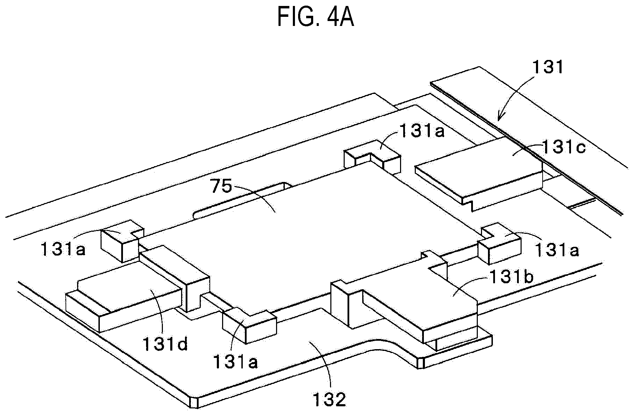

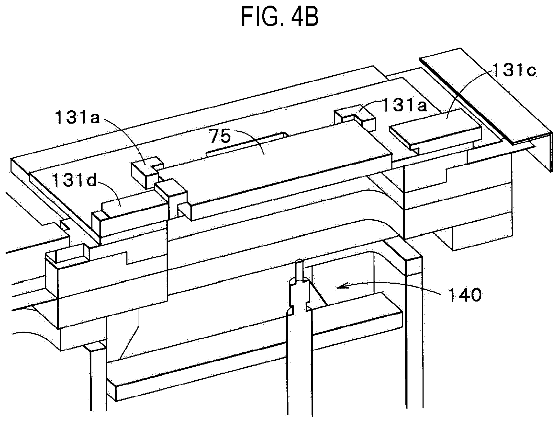

As illustrated in **FIGS. 4A** and **4B****,** the cell removal part 130 includes a container holding part 131 (131a, 131b, 131c and 131d) which holds the second airtight container 75, an XY stage 132 which freely moves the container holding part 131 in the horizontal direction, a cell separation device 140 disposed under the container holding part 131 and the XY stage 132 and configured to separate the colonies of cells adhering to the front surface of the film 77 of the second airtight container 75 by imparting ultrasonic vibration from the rear surface side to the film 77 of the second airtight container 75 held by the container holding part 131, and an elevator part 134 *(see* **FIG**. **3**) which moves the cell separation device 140 in the up-down direction. The elevator part 134 may be a single member which simultaneously and vertically moves the below-mentioned ultrasonic probe 141 and the below-mentioned frame 142 with respect to the rear surface of the film 77. Alternatively, the elevator part 134 may be two members which individually and vertically move the ultrasonic probe 141 and the frame 142 with respect to the rear surface of the film 77.

As illustrated in **FIGS. 4A** and **4B**, the container holding part 131 includes a plurality of (four, in the aspect illustrated in **FIG. 4A**) receiving portions 131a having a substantially L-like cross section, which receive corners of the second airtight container 75 and serve as guides, a pressing portion 131b which presses the second airtight container 75 in the horizontal direction, a lift preventing portion 131c which prevents uplift of the second airtight container 75, and a pressing lift-preventing portion 131d which prevents uplift of the second airtight container 75 while pressing the second airtight container 75 in the horizontal direction. In the present embodiment, the pressing portion 131b is positioned at a long edge side of the second airtight container 75 having a substantially rectangular shape and is configured to press a long edge of the second airtight container 75 in the horizontal direction. Furthermore, the lift preventing portion 131c is positioned at a short edge side (the right side in **FIGS. 4A** and **4B**) of the second airtight container 75 and is configured to prevent lift of a short edge of the second airtight container 75. Moreover, the pressing lift-preventing portion 131d is positioned at a short edge side (the left side in **FIGS. 4A** and **4B**) of the second airtight container 75 and is configured to press a short edge of the second airtight container 75 in the horizontal direction while preventing lift of the short edge of the second airtight container 75.

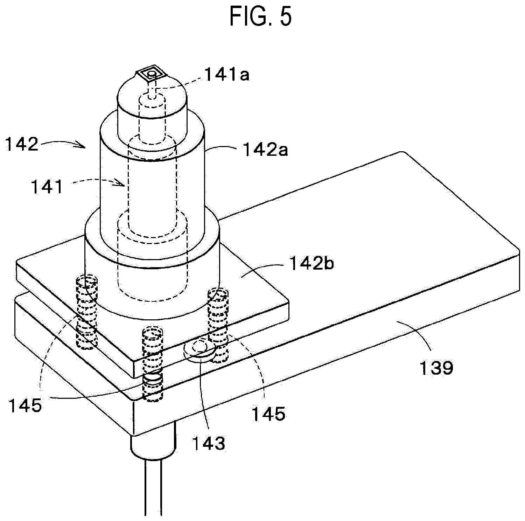

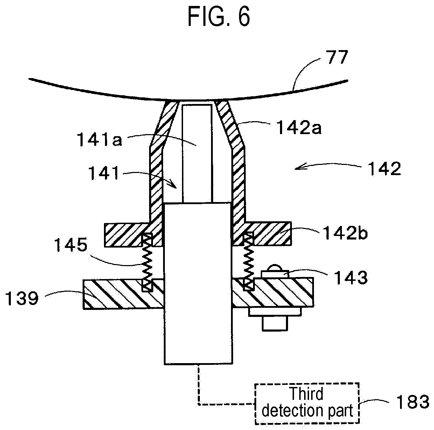

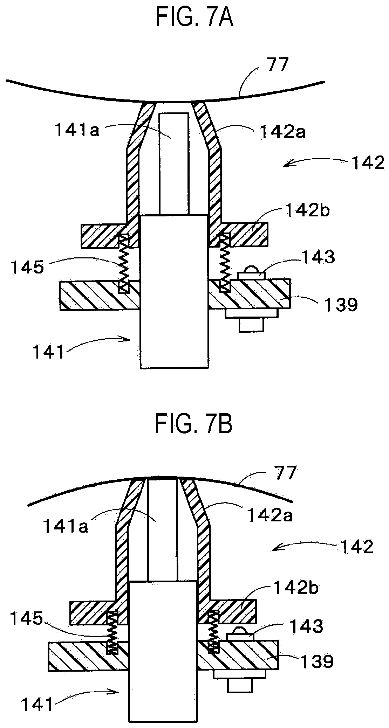

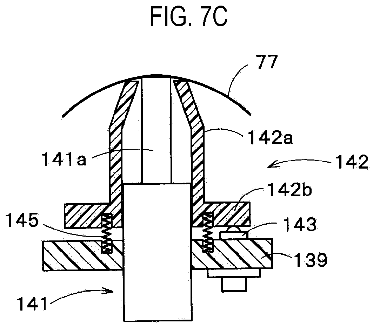

As illustrated in **FIGS. 5** and **6****,** the cell separation device 140 of the present embodiment includes an ultrasonic probe 141 having a vibrator 141a which ultrasonically vibrates in the up-down direction (specified direction), a frame 142 (corresponding to a "vibrator-side frame" of the claims) disposed to surround the ultrasonic probe 141, and a first detection part 143 which detects a force applied to the frame 142 in the up-down direction (specified direction). When the frame 142 does not make contact with the film 77, the end portion of the vibrator 141a existing at the side of the film 77 (the upper end of the vibrator 141a in the present embodiment) is accommodated within the frame 142 *(see* **FIG. 7A**). If the frame 142 is pressed against the film 77, the end portion of the vibrator 141a existing at the side of the film 77 (the upper end of the vibrator 141a in the present embodiment) protrudes upward from the frame 142 (*see* **FIG. 7C****).**

As illustrated in **FIG. 5**, the frame 142 of the present embodiment is disposed so as to surround the vibrator 141a which vibrates in the up-down direction. If the ultrasonic probe 141 is moved upward by the elevator part 134 in a state in which the container holding part 131 holds the second airtight container 75, the frame 142 makes contact with the rear surface of the film 77 (see **FIGS. 7A** to **7C****).** Furthermore, the frame 142 includes a vertical extension portion 142a having a distal end which makes contact with the rear surface of the film 77, and a horizontal extension portion 142b extending from the lower end of the vertical extension portion 142a in the horizontal direction. In the present embodiment, a substantially rectangular probe holding part 139 which holds the peripheral edge of the ultrasonic probe 141 is provided under the horizontal extension portion 142b. A plurality of (four, in the present embodiment) elastic members 145 formed of springs or the like is provided between the horizontal extension portion 142b of the frame 142 and the probe holding part 139. When the frame 142 makes contact with the film 77 and then moves downward, an upward biasing force is applied to the frame 142 by the elastic members 145. Although only three elastic members 145 are illustrated in **FIG. 5**, one elastic member 145 is provided at the back side of the frame 142.

Furthermore, the first detection part 143 described above is provided in the probe holding part 139. The first detection part 143 is positioned under the horizontal extension portion 142b of the frame 142. Thus, in the case where a contact sensor is employed as the first detection part 143, the lower surface of the horizontal extension portion 142b of the frame 142 can make contact with the upper end of the contact sensor. At the time point at which the upper end of the contact sensor makes contact with the lower surface of the horizontal extension portion 142b of the frame 142, the upward movement of the ultrasonic probe 141 caused by the elevator part 134 is stopped. Furthermore, in the case where a proximity sensor is used as the first detection part 143, it is possible to measure the distance between the lower surface of the horizontal extension portion 142b of the frame 142 and the upper end of the proximity sensor. At the time point at which the distance between the lower surface of the horizontal extension portion 142b of the frame 142 and the upper end of the proximity sensor becomes a predetermined distance, the upward movement of the ultrasonic probe 141 caused by the elevator part 134 is stopped. That is to say, in the case where the contact sensor is employed as the first detection part 143, it is determined that a force having a specified magnitude is applied from the rear surface of the film 77 to the frame 142 at the time point at which the upper end of the contact sensor makes contact with the rear surface of the horizontal extension portion 142b of the frame 142. In the case where the proximity sensor is employed as the first detection part 143, it is determined that a force having a specified magnitude is applied from the rear surface of the film 77 to the frame 142 at the time point at which the distance between the upper end of the proximity sensor and the rear surface of the horizontal extension portion 142b of the frame 142 becomes a predetermined distance. Furthermore, in the case where a force sensor is employed as the first detection part 143, it is possible to measure a force applied from the lower surface of the horizontal extension portion 142b of the frame 142 to the upper end of the force sensor. At the time point at which the force applied from the lower surface of the horizontal extension portion 142b of the frame 142 to the upper end of the force sensor becomes a specified magnitude, the upward movement of the ultrasonic probe 141 caused by the elevator part 134 is stopped. That is to say, in the case where the contact sensor is used as the first detection part 143, it is determined that a force having a specified magnitude is applied from the rear surface of the film 77 to the frame 142 at the time point at which the upper end of the contact sensor makes contact with the rear surface of the horizontal extension portion 142b of the frame 142. In the case where the proximity sensor is employed as the first detection part 143, it is determined that a force having a specified magnitude is applied from the rear surface of the film 77 to the frame 142 at the time point at which the distance between the upper end of the proximity sensor and the rear surface of the horizontal extension portion 142b of the frame 142 becomes a predetermined distance. In the case where the force sensor is employed as the first detection part 143, it is determined that a force having a specified magnitude is applied from the rear surface of the film 77 to the frame 142 at the time point at which the force having the specified magnitude is applied from the rear surface of the horizontal extension portion 142b of the frame 142 to the upper end of the force sensor. Incidentally, the aspect illustrated in **FIGS. 7A** to **7C** is directed to a case where the contact sensor is employed as the first detection part 143. Even when the contact sensor is employed as the first detection part 143, it is not necessarily required to stop the upward movement of the ultrasonic probe 141 caused by the elevator part 134 at the time point at which the upper end of the contact sensor makes contact with the rear surface of the horizontal extension portion 142b of the frame 142. Alternatively, the upward movement of the ultrasonic probe 141 may be stopped after the ultrasonic probe 141 is pushed upward by a predetermined amount from the location at which the upper end of the contact sensor makes contact with the rear surface of the horizontal extension portion 142b of the frame 142.

As illustrated in **FIG. 5**, the horizontal cross section of the upper end of the frame 142 of the present embodiment has a rectangular shape. However, the present disclosure is not limited thereto. The horizontal cross section of the upper end of the frame 142 may have different shapes. As one example, the horizontal cross section of the upper end of the frame 142 may have a circular shape. It may be possible to employ an aspect in which four pins positioned in the corners of a rectangle protrude upward and make contact with the rear surface of the film 77. In addition, by employing the frame 142 that has a rectangular horizontal cross section at an upper end thereof as illustrated in **FIG. 5**, it is possible to effectively prevent the vibration of the film 77 caused by the vibration of the vibrator 141 a from spreading outward of the peripheral edge of the frame 142. Examples of the material of the frame 142 may include polyacetal (POM), hard plastic, rubber and the like. By way of example, the cross section of the distal end of the vibrator 141a has a circular shape with the diameter thereof being about 2 mm and the cross section of the distal end of the frame 142 has a square shape with the length of one side thereof being about 3 mm to 4 mm.

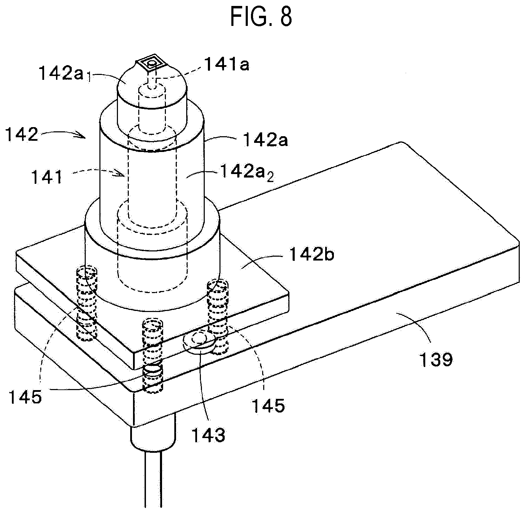

Furthermore, the frame 142 may be formed of two or more parts. For example, as illustrated in **FIG. 8**, the frame 142 may include a first frame 142a1 which makes contact with the rear surface of the film 77 and a second frame 142a2 which is positioned at the lower side of the first frame 142a1 (at the base end side or at the side of the first detection part 143).

The frame 142 of the present embodiment is pushed until the force applied from the rear surface of the film 77 to the frame 142 reaches a specified magnitude. After the frame 142 is pushed until the force applied from the rear surface of the film 77 to the frame 142 has the specified magnitude, the ultrasonic probe 141 is moved upward and the upper end of the vibrator 141a is selectively brought into contact with a portion of the rear surface of the film 77 by a specified force. The vibrator 141a is ultrasonically vibrated in the up-down direction, whereby the colonies of defective cells are separated from the front surface of the film 77.

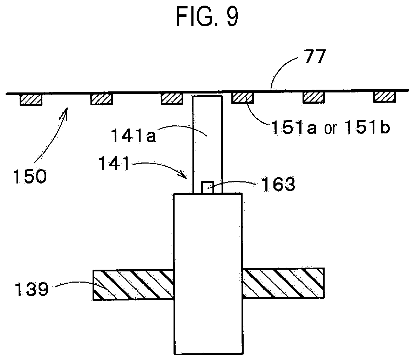

While the first aspect and the second aspect of the present disclosure have been described above by way of example, the present invention is not limited thereto. It may be possible to employ an aspect (third aspect) in which a second detection part 163 (*see* **FIG. 9**) for detecting the force applied from the rear surface of the film 77 to the ultrasonic probe 141 or the distance between the vibrator 141a and the rear surface of the film 77 is provided. Based on the detection result obtained by the second detection part 163, the vibrator 141a is pushed until the force applied from the rear surface of the film 77 to the vibrator 141a reaches a specified magnitude. Thereafter, the vibrator 141a making contact with the rear surface of the film 77 is ultrasonically vibrated. Furthermore, it may be possible to employ an aspect (fourth aspect) in which the frame 142 of the third aspect is employed. Based on the detection result obtained by the second detection part 163, the vibrator 141a is pushed until the force applied from the rear surface of the film 77 to the vibrator 141a reaches a specified magnitude. Thereafter, the vibrator 141 a making contact with the rear surface of the film 77 is ultrasonically vibrated in a state in which the frame 142 makes contact with the rear surface of the film 77.

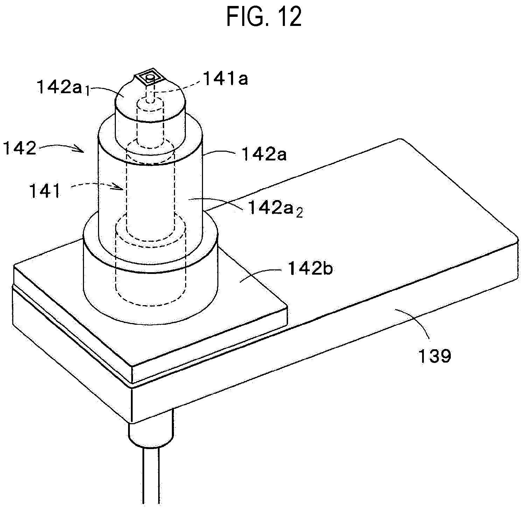

In the case of employing the aspect in which the frame 142 includes the first frame 142a1 and the second frame 142a2 as illustrated in **FIG. 8**, the elastic modulus of the first frame 142a1 may be smaller than the elastic modulus of the second frame 142a2. Specifically, for example, the first frame 142a1 may be formed of an elastic body (a rubber material or the like) and the second frame 142a2 may be made of hard plastic. In this aspect, when the frame 142 makes contact with the film 77, the first frame 142a1 is sandwiched and contracted between the film 77 and the second frame 142a2. The state in which the frame 142 makes contact with the rear surface of the film 77 can be reliably maintained by the restoring force of the first frame 142a1. That is to say, the first frame 142a1 can have the same function as the elastic members 145. Thus, in this embodiment, as illustrated in **FIG. 12**, the elastic members 145 may be removed from between the frame 142 and the probe holding part 139. The frame 142 and the probe holding part 139 may be integrated (fixedly connected).

Instead of the second detection part 163 or in addition to the first detection part 143, there may be provided a third detection part 183 which detects the output power of the ultrasonic probe 141 (*see* **FIG. 6**)**.** By pressing the ultrasonic probe 141 against the rear surface of the film 77 while monitoring the output power of the ultrasonic probe 141 with the third detection part 183 in this way, it is possible to measure and control the pressing distance of the ultrasonic probe 141 with respect to the film 77. In the case of employing the third detection part 183, it is possible to control the pressing distance of the ultrasonic probe 141 with respect to the film 77 without having to use other detection parts such as the first detection part 143 and the second detection part 163 and, ultimately, to control the range of cells separated from the film 77. The ultrasonic probe 141 may be moved upward while continuously vibrating the vibrator 141a. Alternatively, the vibrator 141a may be vibrated only when detecting the output power of the ultrasonic probe 141 and may not be vibrated in other cases, for example, when the ultrasonic probe 141 is moved upward.

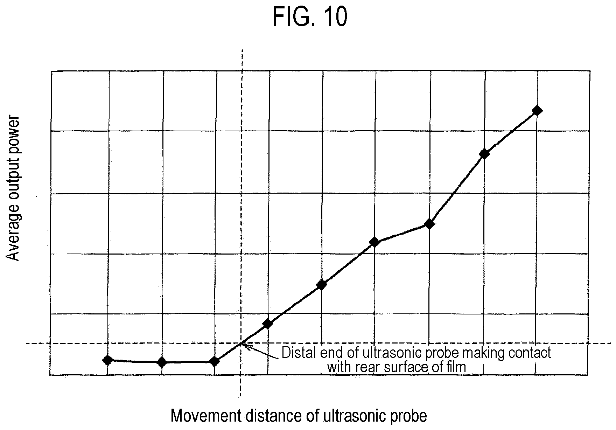

In addition, when the ultrasonic probe 141 is pressed against the film 77 while keeping the output power constant, the range of cells separated from the film 77 varies depending on the pressing distance of the ultrasonic probe 141. Specifically, as the pressing distance becomes larger, the range of cells separated from the film 77 grows wider. Furthermore, when pressing the ultrasonic probe 141 against the film 77, the output power of the ultrasonic probe 141 also varies depending on the pressing distance of the ultrasonic probe 141 (*see* **FIG. 10**). Specifically, as the pressing distance becomes larger, the output power of the ultrasonic probe 141 grows larger. Thus, by pressing the ultrasonic probe 141 against the rear surface of the film 77 while monitoring the output power of the ultrasonic probe 141 with the third detection part 183, it is possible to control the range of cells separated from the film 77. The third detection part 183 is configured to detect the energy irradiated from and reflected to the ultrasonic probe 141, which varies when the distal end of the ultrasonic probe 141 makes contact with the rear surface of the film 77. **FIG. 10** is a graph in which the up-down direction movement distance of the ultrasonic probe 141 is indicated in a horizontal axis and the average output power of the ultrasonic probe 141 is indicated in a vertical axis when a value obtained by dividing the energy consumed in the ultrasonic probe 141 by the process time (e.g., 5 seconds) is referred to as average output power. In the graph illustrated in **FIG. 10**, the distal end of the ultrasonic probe 141 makes contact with the rear surface of the film 77 between the third plot and the fourth plot. As illustrated in **FIG. 10**, the average output power is substantially constant before the distal end of the ultrasonic probe 141 makes contact with the rear surface of the film 77. However, after the distal end of the ultrasonic probe 141 makes contact with the rear surface of the film 77, the average output power becomes larger as the ultrasonic probe 141 is moved upward. Similar to the second detection part, the third detection part 183 can detect the force applied from the rear surface of the film 77 to the vibrator 141a or the contact between the vibrator 141a and the rear surface of the film 77 by monitoring the output power of the ultrasonic probe 14.

### «Method»

When the colonies of defective cells adhering to the front surface of the film 77 and not required in culture or subculture are separated by the cell inspection removal part 25 or 35, the following process goes through.

The second airtight container 75 held by the incubator part 27 or 37 is transferred to the cell inspection part 120 by the in-device transfer part 23 or 33 disposed within the device (the iPS cell automatic culture device 20 or the differentiated cell automatic culture device 30).

The colonies of cells formed on the front surface of the film 77 are observed by the optical microscope 121 (*see* FIG. 3) of the cell inspection part 120. Then, the positions of the colonies of cells are recognized based on the image obtained by the optical microscope 121 and the quality of each of the colonies is determined by the determination part 122. In the case where the colonies of defective cells exist, the positions thereof are stored in the inspection memory part 123.

The second airtight container 75 containing defective cells is transferred to the container holding part 131 of the cell removal part 130 by the in-device transfer part 23 or 33 disposed within the device (iPS cell automatic culture device 20 or the differentiated cell automatic culture device 30) (*see* **FIGS. 4A** and **4B**). The second airtight container 75 transferred to the container holding part 131 in this way is positioned inside four receiving portions 131a. Thereafter, the lift preventing portion 131c covers the upper surface of one short edge of the second airtight container 75. The pressing lift-preventing portion 131d covers the upper surface of the other short edge of the second airtight container 75 and presses the side surfaces of the short edges of the second airtight container 75 in the horizontal direction. The pressing portion 131b presses the side surface of the long edge of the second airtight container 75. At this time, the second airtight container 75 is positioned so that the film 77 thereof lies at the lower side of the container body.

Subsequently, the second airtight container 75 is moved by the XY stage 132, whereby the location where the colonies of defective cells to be removed exist is positioned above the vibrator 141a of the ultrasonic probe 141.

If the second airtight container 75 is positioned as above, the ultrasonic probe 141 is moved up by the elevator part 134. If the ultrasonic probe 141 is moved up in this way, the upper end of the vertical extension portion 142a of the frame 142 is first pushed toward the rear surface of the film 77 (*see* **FIG. 7A**). By further moving the ultrasonic probe 141 upward, the elastic members 145 are contracted by the force received from the film 77. The frame 142 is relatively moved downward with respect to the ultrasonic probe 141. The upper end of the vibrator 141a of the ultrasonic probe 141 and the upper end of the frame 142 are positioned on the same plane (*see* **FIG. 7B**)**.** By further moving the ultrasonic probe 141 upward, the upper end of the vibrator 141a of the ultrasonic probe 141 is positioned more upward than the upper end of the frame 142 (*see* **FIG. 7C**). If it is determined based on the detection result of the first detection part 143 that the force applied from the rear surface of the film 77 to the upper ends of the frame 142 and the vibrator 141a reaches a specified magnitude, the upward movement of the ultrasonic probe 141 caused by the elevator part 134 is stopped. Thereafter, the vibrator 141a is ultrasonically vibrated in the up-down direction, whereby ultrasonic vibration is applied to the film 77 of the second airtight container 75 from the rear surface side. Thus, the target defective cells (iPS cells or differentiated cells) are separated from the container-body-side surface.

In the case where a contact sensor is employed as the first detection part 143, the upward movement of the ultrasonic probe 141 caused by the elevator part 134 is stopped at the time point at which the lower surface of the horizontal extension portion 142b of the frame 142 makes contact with the upper end of the contact sensor. On the other hand, in the case where a proximity sensor is employed as the first detection part 143, the upward movement of the ultrasonic probe 141 caused by the elevator part 134 is stopped at the time point at which the distance between the lower surface of the horizontal extension portion 142b of the frame 142 and the upper end of the proximity sensor becomes a predetermined distance.

In the case where a plurality of colonies of defective cells exists in the target second airtight container 75, after the target colony of defective cells is separated from the front surface of the film 77, the second airtight container 75 is moved in the horizontal direction by the XY stage 132, whereby the location where the next colony of defective cells to be removed exists is positioned above the vibrator 141 a. Then, the colony of defective cells is separated from the front surface of the film 77 by the same method as mentioned above. The aforementioned process is repeatedly performed until all the colonies of defective cells to be removed are separated from the front surface of the film 77 of the second airtight container 75.

If all the colonies of defective cells are separated from the front surface of the film 77 of the second airtight container 75 in the aforementioned manner, the second airtight container 75 is transported to the liquid storage supply part 26 or 36 by the in-device transfer part 23 or 33 disposed within the device (the iPS cell automatic culture device 20 or the differentiated cell automatic culture device 30). Thereafter, a liquid culture medium is supplied from the inlet into the second airtight container 75, whereby the separated floating defective cells are pushed out from the second airtight container 75 through the outlet and are discharged from the discharge part 28 or 38. The timing at which the liquid culture medium is replaced in this way may be immediately after the cell removal is performed by the cell inspection removal part 25 or 35 or may be after a certain period of time elapses.

### «Effects»

Next, the effects achieved by the first aspect, the second aspect, the third aspect and the fourth aspect configured as above, which are not yet described but are especially important, will be described.

According to the first aspect, the second aspect, the third aspect and the fourth aspect, the vibrator 141a selectively imparts ultrasonic vibration to the specified cells adhering to the front surface of the film 77 of the second airtight container 75 (*see* **FIG. 7C**). Thus, it is possible to realize the selective separation of the colonies of specified cells such as defective cells or the like by virtue of ultrasonic vibration, which was difficult to realize in the technique disclosed in

### Patent Document 1.

Furthermore, according to the second aspect, the vibrator 141a ultrasonically vibrating in the up-down direction is surrounded by the frame 142. The ultrasonic probe 141 provided with the vibrator 141a and the frame 142 are moved upward by the elevator part 134. The upper ends of the ultrasonic probe 141 and the frame 142 make contact with the rear surface of the film 77. At this time, the force applied in the up-down direction by the frame 142 is detected by the first detection part 143. Then, based on the detection result obtained by the first detection part 143, the frame 142 is pushed until the force applied from the rear surface of the film 77 to the frame 142 reaches a specified magnitude. Thereafter, the upper end of the vibrator 141a makes contact with the rear surface of the film 77 by a predetermined force in a state in which the upper end of the frame 142 makes contact with the rear surface of the film 77. Subsequently, the vibrator 141a is ultrasonically vibrated. Thus, ultrasonic vibration can be imparted to a specified location of the film 77 by the vibrator 141a while pressing the film 77 of the second airtight container 75 from the rear surface side with a force having an appropriate magnitude by virtue of the frame 142. As a result, it is possible to prevent the vibrator 141a from being too strongly pressed against the film 77 and penetrating the film 77. It is also possible to prevent the contact of the vibrator 141a with the film 77 from being insufficient, prevent the vibration applied from the vibrator 141a to the film 77 from becoming uneven and prevent the separation state of the colonies of cells from becoming non-uniform.

According to the first aspect, the second aspect and the fourth aspect, the frame 142 is provided around the vibrator 141a. The frame 142 surrounds the upper end of the vibrator 141a and can support the rear surface of the film 77 of the second airtight container 75 around the upper end of the vibrator 141a. Therefore, as compared with a case where the rear surface of the film 77 is supported by only the vibrator 141 a, it is possible to reduce the pressure applied to the rear surface of the film 77. Furthermore, in the second aspect, not only the frame 142 surrounds the upper end of the vibrator 141a, but the frame 142 can also maintain the force applied from the rear surface of the film 77 to the frame 142 at a specified magnitude based on the detection result obtained by the first detection part 143. Thus, it is possible to prevent the vibrator 141a or the frame 142 from being too strongly pressed against the film 77 and penetrating the film 77. Furthermore, in the second aspect, the vibrator 141a makes contact with the rear surface of the film 77 and ultrasonically vibrates in a state in which the force applied from the rear surface of the film 77 to the frame 142 is maintained at a specified magnitude. Thus, when separating the colonies of cells, it is possible to impart vibration from the vibrator 141a to the rear surface of the film 77 in a state in which the rear surface of the bent film 77 is supported with a substantially uniform force by the upper end of the frame 142 and the upper end of the vibrator 141a. As a result, the upper end of the vibrator 141a makes contact with the rear surface of the film 77 by a force having a specified magnitude in a state in which the downwardly-bent film 77 is supported with a predetermined force by the frame 142. Thereafter, ultrasonic vibration can be imparted to the rear surface of the film 77. Ultimately, it is possible to make uniform the vibration imparted from the vibrator 141 a and to make uniform the separation state of the colony of cells at every separation time.

Furthermore, in the first aspect, the second aspect and the fourth aspect, the vibrator 141a ultrasonically vibrates in a state in which the frame 142 supports the rear surface of the film 77 of the second airtight container 75 around the upper end of the vibrator 141a. Since the upper end of the frame 142 makes contact with the rear surface of the film 77 at the outer side of the peripheral edge of the vibrator 141a in this way, it is possible to prevent the vibration of the film 77 attributable to the vibration of the vibrator 141a from spreading outward of the peripheral edge of the frame 142. Thus, it is possible to limit the influence of the vibration generated by the vibrator 141a to the target colony of defective cells as near as possible. This makes it possible to prevent the colony of good cells adjoining the colony of defective cells from being separated by mistake.

Furthermore, in the aspect illustrated in **FIGS. 7A** to **7C**, when the frame 142 does not make contact with the film 77, the upper end of the vibrator 141 a is accommodated inside the frame 142 (*see* **FIG. 7A**). As the frame 142 is pressed against the film 77, the upper end of the vibrator 141a protrudes upward from the frame 142 (*see* **FIG. 7C****).** Thus, after the upper end of the frame 142 having a large cross-sectional area is first brought into contact with the rear surface of the film 77, the upper end of the vibrator 141a having a small cross-sectional area can be brought into contact with the rear surface of the film 77. This makes it possible to prevent a force from being too strongly applied to a local area of the film 77 and to prevent the film 77 from being broken.

Furthermore, in the case where a contact sensor is employed as the first detection part 143, the upward movement of the ultrasonic probe 141 caused by the elevator part 134 can be stopped at the time point at which the lower surface of the horizontal extension portion 142b makes contact with the upper end of the contact sensor. In the case where a proximity sensor is employed as the first detection part 143, the upward movement of the ultrasonic probe 141 caused by the elevator part 134 can be stopped at the time point at which the distance between the lower surface of the horizontal extension portion 142b and the upper end of the proximity sensor becomes a predetermined distance. Thus, it is possible to stop the upward movement of the ultrasonic probe 141 in a state in which the up-down-direction distance between the horizontal extension portion 142b and the probe holding part 139 is always kept constant. Ultimately, the vibrator 141 a can be caused to ultrasonically vibrate while always keeping constant the position of the vibrator 141a protruding from the upper end of the frame 142. That is to say, in a state in which the vibrator 141a is stopped, it is possible to always keep constant the force acting between the film 77 and the frame 142 and the force acting between the film 77 and the vibrator 141a. Thus, the deflection conditions of the film 77 of the second airtight container 75 with respect to the vibrator 141a and the frame 142 can be kept the same at every separation time. This makes it possible to keep uniform the separation state of the colony including defective cells at every separation time.

In the case of employing the aforementioned third detection part 183 (*see* **FIG. 6**), the ultrasonic probe 141 is pressed against the rear surface of the film 77 while monitoring the output power of the ultrasonic probe 141 with the third detection part 183. Thus, it is possible to measure and control the pressing distance of the ultrasonic probe 141 with respect to the film 77. Ultimately, it is possible to control the range of cells separated from the film 77.

Furthermore, in the case where the frame 142 is formed of hard plastic, rubber or the like and has a large elastic modulus, it is possible for the material having a large elastic modulus to prevent spreading of the vibration of the film 77 attributable to the vibration of the vibrator 141a. Thus, it is possible to reliably limit the influence of the vibration caused by the vibrator 141 a to the target colony of defective cells. This makes it possible to reliably prevent the colony of good cells adjoining the colony of defective cells from being separated by mistake. The same effects can be achieved in the case where the aspect in which the frame 142 includes the first frame 142a1 and the second substrate 12 as illustrated in **FIG. 8** is employed and in the case where the first frame 142a1 is formed of hard plastic, rubber or the like and the first frame 142a1 has a large elastic modulus.

Furthermore, in the aspect illustrated in **FIG. 5**, four elastic members 145 are disposed at regular intervals. Therefore, the frame 142 making contact with the rear surface of the film 77 is not tilted in the biased direction. It is possible to press the rear surface of the film 77 in the direction conforming to the normal direction of the bent film 77 as near as possible. Thus, the deflection conditions of the film 77 of the second airtight container 75 with respect to the vibrator 141a and the frame 142 can be kept the same at every separation time. Ultimately, the separation state of the colony including defective cells can be kept uniform at every separation time.

The fifth aspect and the sixth aspect employ a frame 150 that makes contact with the rear surface of the film 77 at the outer side of the peripheral edge of the vibrator 141a when the vibrator 141a is brought into contact with the rear surface of the film 77. The frame 150 is disposed to surround the target range of a separation operation on the rear surface of the film 77. When the second airtight container 75 is held by the container holding part 131, the frame 150 may be mounted under the second airtight container 75 and may be held together with the second airtight container 75 by the container holding part 131. Alternatively, the frame 150 may be bonded to each of the second airtight containers 75 in advance.

Furthermore, in the fifth aspect and the sixth aspect, instead of the first detection part 143 for detecting the force applied from the rear surface of the film 77 to the frame or the distance between the frame and the rear surface of the film 77, there may be provided a second detection part 163 for detecting the force applied from the rear surface of the film 77 to the vibrator 141a or the distance between the vibrator 141a and the rear surface of the film 77 (*see* **FIG. 9**)*.* Based on the detection result obtained by the second detection part 163, the vibrator 141a is pushed until the force applied from the rear surface of the film 77 to the vibrator 141a reaches a specified magnitude. Thereafter, the vibrator 141a ultrasonically vibrates in a state in which the frame 150 makes contact with the rear surface of the film 77.

In the fifth aspect and the sixth aspect, the container holding part 131 holds the second airtight container 75 and the frame 150, whereby the frame 150 is bought into contact with the rear surface of the film 77. Then, the vibrator 141a is pushed against the film 77 through a gap provided in the frame 150. At this time, based on the detection result obtained by the second detection part 163, the vibrator 141a is pushed against the rear surface of the film 77 until the force applied from the rear surface of the film 77 to the vibrator 141a reaches a specified magnitude. Then, the vibrator 141a making contact with the rear surface of the film 77 ultrasonically vibrates in a state in which the frame 150 makes contact with the rear surface of the film 77. At this time, the vibrator 141a selectively imparts ultrasonic vibration to the specified cells adhering to the front surface of the film 77.

In the fifth aspect and the sixth aspect, other configurations are substantially the same as those of the second aspect. In the fifth aspect and the sixth aspect, the same parts as those of the second aspect will be designated by like reference numerals with the detailed descriptions thereof omitted.

In fifth aspect and the sixth aspect, it is possible to achieve the same effects as those described in the first aspect, the second aspect, the third aspect and the fourth aspect. Hereinafter, the important effects achieved in the fifth aspect and the sixth aspect will be described.

According to the fifth aspect and the sixth aspect, the vibrator 141a selectively imparts ultrasonic vibration to the specified cells adhering to the front surface of the film 77 of the second airtight container 75 (*see* **FIG. 7C**). Thus, it is possible to realize the region-selective separation of the colony of specified cells such as defective cells or the like by virtue of ultrasonic vibration, which was difficult to realize in the technique disclosed in Patent Document 1.

Furthermore, according to the sixth aspect, the vibrator 141a ultrasonically vibrating in the up-down direction is surrounded by the frame units 151a and 151b of the frame 150. The ultrasonic probe 141 provided with the vibrator 141a is moved upward by the elevator part 134. The upper end of the ultrasonic probe 141 makes contact with the rear surface of the film 77. At this time, the up-down-direction force applied to the vibrator 141 a is detected by the second detection part 163. Then, based on the detection result obtained by the second detection part 163, the vibrator 141a is pushed until the force applied from the rear surface of the film 77 to the vibrator 141a reaches a specified magnitude. Thereafter, the upper end of the vibrator 141a makes contact with the rear surface of the film 77 by a predetermined force in a state in which the frame 150 makes contact with the rear surface of the film 77. In this state, the vibrator 141a is ultrasonically vibrated. Thus, ultrasonic vibration can be imparted to a specified location of the film 77 by the vibrator 141a after the film 77 of the second airtight container 75 is pressed from the rear surface side with a force having an appropriate magnitude by virtue of the vibrator 141a. As a result, it is possible to prevent the vibrator 141a from being too strongly pressed against the film 77 and penetrating the film 77. It is also possible to prevent the contact of the vibrator 141a with the film 77 from being insufficient, prevent the vibration applied from the vibrator 141a to the film 77 from becoming uneven and prevent the separation state of the colonies of cells from becoming non-uniform.

Descriptions will be made on this point. According to the sixth embodiment, the frame units 151a and 151b of the frame 150 are disposed around the vibrator 141a. The frame units 151a and 151b of the frame 150 surround the upper end of the vibrator 141a and can support the rear surface of the film 77 of the second airtight container 75 around the upper end of the vibrator 141a. Thus, as compared with a case where the rear surface of the film 77 is supported by only the vibrator 141a, it is possible to reduce the pressure applied to the rear surface of the film 77. Furthermore, in the sixth aspect, based on the detection result obtained by the second detection part 163, the force applied from the rear surface of the film 77 to the vibrator 141a can be maintained at a specified magnitude. This makes it possible to prevent the vibrator 141a from being too strongly pressed against the film 77 and penetrating the film 77. Furthermore, in the sixth aspect, after the force applied from the rear surface of the film 77 to the vibrator 141a is kept at a specified magnitude, the vibrator 141a makes contact with the rear surface of the film 77 and ultrasonically vibrates. Thus, each time when the colony of cells is separated, it is possible to impart vibration from the vibrator 141a to the rear surface of the film 77 with a substantially uniform force. As a result, it is possible to impart ultrasonic vibration to the rear surface of the film 77 after the upper end of the vibrator 141a makes contact with the rear surface of the film 77 by the force having a specified magnitude. Ultimately, it is possible to make uniform the vibration imparted from the vibrator 141 a and to make uniform the separation state of the colony of cells at every separation time.

Furthermore, in the fifth aspect and the sixth aspect, the vibrator 141a ultrasonically vibrates in a state in which the frame 150 supports the rear surface of the film 77 of the second airtight container 75 around the upper end of the vibrator 141a. Since the upper end of the frame 142 makes contact with the rear surface of the film 77 at the outer side of the peripheral edge of the vibrator 141a in this way, it is possible to prevent the vibration of the film 77 attributable to the vibration of the vibrator 141a from spreading to the outer side of the peripheral edge of the frame 142. Thus, it is possible to limit the influence of the vibration generated by the vibrator 141 to the specified target colony of defective cells as near as possible. This makes it possible to prevent the colony of good cells adjoining the colony of defective cells from being separated by mistake.

In the respective aspects described above, it is preferred that the film 77 of the second airtight container 75 is made of a thermosetting resin material, especially thermosetting rubber (e.g., silicon rubber). In the case where the film 77 is made of a thermoplastic resin material (e.g., a polystyrene film), the region electivity becomes poor. Thus, there is a possibility that not only the colony of cells existing in the contact region of the vibrator 141a is separated, but also the colony of good cells existing around the contact region is separated. Furthermore, if the vibration time of the vibrator 141a is too long or if the pressing force is too strong, there is a possibility that the film 77 is melted by friction heat, a hole is formed in the film 77 and liquid leaks. On the other hand, in the case where the film 77 is made of a thermosetting resin material, especially thermosetting rubber (e.g., silicon rubber), the region selectivity is improved. Thus, there is no possibility that the film 77 is melted by friction heat and a hole is formed in the film 77.

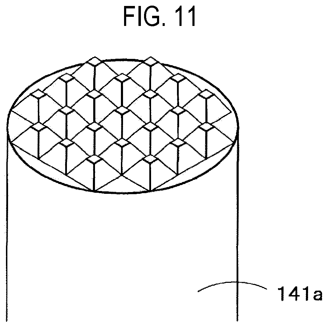

As illustrated in **FIG. 11**, it is preferred that the end portion of the vibrator 141a existing at the side of the film 77 is subjected to knurling process. In the case where the end portion of the vibrator 141a existing at the side of the film 77 is formed in a planar shape, the ultrasonic vibration energy in the central region of the end portion becomes more stronger than the ultrasonic vibration energy in the peripheral region of the end portion. On the other hand, in the case where the end portion of the vibrator 141a existing at the side of the film 77 is subjected to a knurling process, it is possible to restrain the ultrasonic vibration energy from being concentrated on the central region of the end portion and to make uniform the distribution of the ultrasonic vibration energy in the end portion. Thus, when the vibrator 141 a is brought into contact with the rear surface of the film 77, it is possible to restrain the ultrasonic vibration energy from being excessively supplied to a portion of the contact range. This makes it possible to further reduce the risk that the film 77 is broken.

The present inventor prepared OptiCell (trademark) in which silicon rubber of 0.05 mm in thickness is used as a base material of the film 77. The knurled distal end of the vibrator 141a of 1.8 mm in diameter was brought into contact with the rear surface of the film 77 without using the frame 142 or 150. The vibrator 141a was vibrated at 70 kHz for 2 seconds. As a result, it was possible to reduce an effective separation region to the diameter (1.8 mm) of the distal end of the vibrator 141a plus 0.8 mm. If it is considered that a colony of iPS cells is preferably cultured at a diameter of about 3.0 mm or less in order to maintain non-differentiation, it is preferred that the diameter of the distal end of the vibrator 141a is 2.2 mm (3 mm - 0.8 mm) or less. Furthermore, if it is considered that a method of culturing a colony of iPS cells at a diameter of 1 mm or more is widely used, it is preferred that the diameter of the distal end of the vibrator 141a is 1 mm or more. Furthermore, the present inventor caused the vibrator 141a to vibrate in a state in which the vibrator 141a is pushed into the rear surface of the film 77 by 1 mm. Even in this case, the film 77 was not broken. Moreover, the present inventor caused the vibrator 141a to vibrate at 70 kHz for 10 seconds in a state in which the vibrator 141a is brought into contact with the rear surface of the forme 77. Even in this case, the film 77 was not broken.

Next, the present inventor prepared OptiCell (trademark) in which a polystyrene of 0.07 mm in thickness is used as a base material of the film 77. The distal end of the vibrator 141a of 2 mm in diameter, which is not subjected to a knurling process, was brought into contact with the rear surface of the film 77 without using the frame 142 or 150. The vibrator 141a was vibrated at 70 kHz for 2 seconds. As a result, the effective separation region was 5 mm or more in diameter. Furthermore, the present inventor caused the vibrator 141a to vibrate at 70 kHz for 5 seconds in a state in which the vibrator 141a is brought into contact with the rear surface of the film 77. As a result, the film 77 was melted by heat and a hole was formed in the film 77. Thus, the culture medium leaked.

Finally, the foregoing descriptions of the respective embodiments and the disclosure of the drawings are nothing more than one example for describing the present disclosure recited in the claims. The present disclosure recited in the claims shall not be limited by the foregoing descriptions of the respective embodiments and the disclosure of the drawings.

### EXPLANATION OF REFERENCE NUMERALS

75: second airtight container, 77: film (cell culture film), 140: cell separation device, 141: ultrasonic probe, 141a: vibrator, 142: frame, 142a1: first frame, 142a2: second frame, 143: first detection part, 145: elastic member, 150: frame, 151a: frame unit, 151b: frame unit

## Claims

1. A cell separation device for imparting vibration to a cell culture film having a front surface to which cells adhere, from a rear surface side of the cell culture film, and consequently separating the cells from the cell culture film, comprising:
an ultrasonic probe having an ultrasonically-vibrating vibrator,
wherein the vibrator selectively making contact with a portion of a rear surface of the cell culture film is ultrasonically vibrated.

2. The device of Claim 1, further comprising:
a second detection part configured to detect a force applied from the rear surface of the cell culture film to the vibrator or a distance between the vibrator and the rear surface of the cell culture film,
wherein based on a detection result obtained by the second detection part, the vibrator is pressed against the rear surface of the cell culture film until the force applied from the rear surface of the cell culture film to the vibrator reaches a specified magnitude, and then the vibrator making contact with the rear surface of the cell culture film is ultrasonically vibrated.

3. The device of Claim 1 or 2, further comprising:
a cell-culture-film-side frame disposed to surround a range becoming a separation operation target or a vibrator-side frame disposed to surround the vibrator,
wherein the vibrator making contact with the rear surface of the cell culture film is ultrasonically vibrated to separate the cells from the cell culture film, and
the cell-culture-film-side frame or the vibrator-side frame always makes contact with the rear surface of the cell culture film while separating the cells from the cell culture film.

4. The device of Claim 1 or 2, further comprising:
a vibrator-side frame disposed to surround the vibrator,
wherein the vibrator-side frame is first pressed against the rear surface of the cell culture film, and then the vibrator making contact with the rear surface of the cell culture film is ultrasonically vibrated in a state in which the vibrator-side frame makes contact with the rear surface of the cell culture film.

5. The device of Claim 4, further comprising:
a first detection part configured to detect a force applied from the rear surface of the cell culture film to the vibrator-side frame or a distance between the vibrator-side frame and the rear surface of the cell culture film,
wherein based on a detection result obtained by the first detection part, the vibrator-side frame is pressed against the rear surface of the cell culture film until the force applied from the rear surface of the cell culture film to the vibrator-side frame reaches a specified magnitude, and then the vibrator making contact with the rear surface of the cell culture film is ultrasonically vibrated in a state in which the vibrator-side frame makes contact with the rear surface of the cell culture film.

6. The device of any one of Claims 3 to 5, wherein the ultrasonic probe and the vibrator-side frame are connected to each other through an elastic member,
a cell-culture-film-side end portion of the vibrator is accommodated inside the vibrator-side frame when the frame does not make contact with the cell culture film, and
the cell-culture-film-side end portion of the vibrator protrudes out of the vibrator-side frame as the vibrator-side frame is pressed against the cell culture film.

7. The device of Claim 2 or 5, wherein the first detection part or the second detection part is a contact sensor, a proximity sensor or a force sensor.

8. The device of any one of Claims 3 to 6, wherein the vibrator-side frame includes a first frame making contact with the rear surface of the cell culture film and a second frame positioned at a side of a base end portion of the first frame, and
an elastic modulus of the first frame is larger than an elastic modulus of the second frame.

9. The device of any one of Claims 3 to 6, wherein the vibrator-side frame includes a first frame making contact with the rear surface of the cell culture film and a second frame positioned at a side of a base end portion of the first frame, and
an elastic modulus of the first frame is smaller than an elastic modulus of the second frame.

10. The device of any one of Claims 4, 5, 6, 8 and 9, further comprising:
one elevator part configured to simultaneously and vertically drive the ultrasonic probe and the vibrator-side frame with respect to the rear surface of the cell culture film.

11. The device of any one of Claims 1 to 10, wherein a cell-culture-film-side end portion of the vibrator is subjected to a knurling process.

12. The device of any one of Claims 1 to 11, wherein the cells separated from the cell culture film are defective cells not required in culture or subculture.

13. The device of any one of Claims 1 to 12, wherein the cells adhering to the front surface of the cell culture film are iPS cells or differentiated cells.

14. A cell separation method for imparting vibration to a cell culture film having a front surface to which cells adhere, from a side of a rear surface
of the cell culture film, and consequently separating the cells from the cell culture film, comprising:
a step of ultrasonically vibrating a vibrator which selectively makes contact with a portion of a rear surface of the cell culture film,
wherein the vibrator is configured to selectively impart ultrasonic vibration to specified cells adhering to the front surface of the cell culture film.

15. The method of Claim 14, further comprising:
a step of bringing the vibrator into contact with the rear surface of the cell culture film, and
a step of, based on a detection result obtained by a second detection part for detecting a force applied from the rear surface of the cell culture film to the vibrator or a distance between the vibrator and the rear surface of the cell culture film, pressing the vibrator against the rear surface of the cell culture film until the force applied from the rear surface of the cell culture film to the vibrator reaches a specified magnitude.

16. The method of Claim 14, further comprising:
a step of bringing a vibrator-side frame or a cell-culture-film-side frame into contact with the rear surface of the cell culture film;
a step of bringing the vibrator into contact with the rear surface of the cell culture film, and
a step of, based on a detection result obtained by a second detection part for detecting a force applied from the rear surface of the cell culture film to the vibrator or a distance between the vibrator and the rear surface of the cell culture film, pressing the vibrator against the rear surface of the cell culture film until the force applied from the rear surface of the cell culture film to the vibrator reaches a specified magnitude,
wherein the step of ultrasonically vibrating the vibrator which makes contact with the rear surface of the cell culture film is performed in a state in which the vibrator-side frame or the cell-culture-film-side frame makes contact with the rear surface of the cell culture film.

17. The method of Claim 14, further comprising:
a step of disposing a cell-culture-film-side frame to surround a range becoming a separation operation target;
a step of pressing the vibrator against the rear surface of the cell culture film; and
a step of, based on a detection result obtained by a second detection part for detecting a force applied from the rear surface of the cell culture film to the vibrator or a distance between the vibrator and the rear surface of the cell culture film, pressing the vibrator against the rear surface of the cell culture film until the force applied from the rear surface of the cell culture film to the vibrator reaches a specified magnitude,
wherein the step of ultrasonically vibrating the vibrator which makes contact with the rear surface of the cell culture film is performed in a state in which the cell-culture-film-side frame makes contact with the rear surface of the cell culture film.

18. The method of Claim 14, further comprising:
a step of bringing a vibrator-side frame into contact with the rear surface of the cell culture film;
a step of, based on a detection result obtained by a first detection part for detecting a force applied from the rear surface of the cell culture film to the vibrator-side frame or a distance between the vibrator-side frame and the rear surface of the cell culture film, pressing the vibrator-side frame against the rear surface of the cell culture film until the force applied from the rear surface of the cell culture film to the vibrator-side frame reaches a specified magnitude; and
a step of pressing the vibrator against the rear surface of the cell culture film,
wherein the step of ultrasonically vibrating the vibrator which makes contact with the rear surface of the cell culture film is performed in a state in which the vibrator-side frame makes contact with the rear surface of the cell culture film.
